# EUROPEAN PATENT SPECIFICATION

(11) **EP 4 034 674 B1**
(45) Date of publication and mention of the grant of the patent: **26.03.2025**
(21) Application number: 20772085.5
(22) Date of filing: 22.09.2020
(51) Int. Cl.: C12Q 1/6818, C12Q 1/6851

(54) **PROBE AND METHOD FOR STR-GENOTYPING**
SONDE UND VERFAHREN ZUR STR-GENOTYPISIERUNG
SONDE ET PROCÉDÉ DE GÉNOTYPAGE STR

(30) Priority: 23.09.2019 EP 19199001
(43) Date of publication of application: 03.08.2022
(73) Proprietor: Universiteit Gent, 9000 Gent (BE)
(72) Inventor: TYTGAT, Olivier, 9090 Melle (BE); VAN NIEUWERBURGH, Filip, 9820 Merelbeke (BE); DEFORCE, Dieter, 8520 Kuurne (BE); CORNELIS, Senne, 9070 Destelbergen (BE)
(86) International application number: PCT/EP2020/076410
(87) International publication number: WO 2021/058470

(56) References cited:
- WO-A1-2009/049253
- WO-A2-02/14555
- WO-A2-2005/113822
- WO-A2-2010/060046
- MAO HUIHUI ET AL: "The mechanism and regularity of quenching the effect of bases on fluorophores: the base-quenched probe method.", THE ANALYST 09 JUL 2018, vol. 143, no. 14, 9 July 2018 (2018-07-09), pages 3292 - 3301, XP002798411, ISSN: 1364-5528
- FRENCH D J ET AL: "Interrogation of short tandem repeats using fluorescent probes and melting curve analysis: A step towards rapid DNA identity screening", FORENSIC SCIENCE INTERNATIONAL: GENETICS, ELSEVIER BV, NETHERLANDS, vol. 2, no. 4, 1 September 2008 (2008-09-01), pages 333 - 339, XP023438759, ISSN: 1872-4973, [retrieved on 20080610], DOI: 10.1016/J.FSIGEN.2008.04.007
- MICAH D. HALPERN ET AL: "An STR Melt Curve Genotyping Assay for Forensic Analysis Employing an Intercalating Dye Probe FRET* : STR FRET MELT CURVE GENOTYPING ASSAY", JOURNAL OF FORENSIC SCIENCES, vol. 56, no. 1, 14 September 2010 (2010-09-14), CHICAGO, IL, US, pages 36 - 45, XP055728133, ISSN: 0022-1198, DOI: 10.1111/j.1556-4029.2010.01549.x

## Description

### Technical field of the invention

The present invention relates to the field of DNA-fingerprinting, e.g. in a forensic setting. More specifically, the present invention discloses a probe and method to genotype polymorphous short tandem repeats, relying on the fluorescence-quenching properties of some nucleotides on some specific fluorophores. As such, the extent of complementarity between an amplified DNA sample and a specifically designed probe can be assessed by measuring the fluorescence intensity of the fluorophore attached to the probe upon hybridization or melting. The probes and method of the present invention are well-suited to be used in a portable, less-expensive DNA-analysis device and can be applied in other fields than forensics, like food fraud, diagnostics and many others.

### Background of the invention

In order to identify individuals based on DNA evidence, a process called DNA-fingerprinting, one must analyze DNA polymorphisms, e.g. single nucleotide polymorphisms (SNPs) or short tandem repeats (STRs). Individuals can denote humans, but also animals and other DNA-containing species.

Polymorphisms in the genome contain an enormous amount of information. The most common type of genetic polymorphisms is classified as SNPs. The presence of a SNP at a certain position in the genome indicates that, within a certain population, the same nucleotide does not occur at this specific position in every individual of this population. SNPs can be bi-allelic, indicating that 2 possible nucleotides occur at this position within a certain population. Recently, more and more notice is taken of the existence of tri- or tetra-allelic SNPs, which have more discriminative power than bi-allelic SNPs [1]. A variant caused by insertion or deletion of one nucleotide at a certain position in the genome is sometimes also denoted as a SNP, but is also known as an indel (short for 'insertion/deletion').

Forensic DNA genotyping is nowadays almost exclusively done by examining STRs. These are regions of short sequences (a few nucleotides, typically 4) which are repeated several times. STR-regions are polymorphous pertaining to the number of repeats, which on its turn defines the possible alleles of a certain STR-region [2].

In the human genome, multiple regions containing this specific type of polymorphism are identified. A statistically unique profile is obtained by analyzing a large number of STR-loci, mostly located in the noncoding regions of the human genome for forensic purposes. In Europe, typically a panel of 12 STRs was examined, called the European Standard Set (ESS). This panel is now expanded with 5 additional loci [3]. In the US, the Combined DNA Index System (CODIS) is used, containing 13 core loci and 7 additional loci [4].

After amplification by the polymerase chain reaction (PCR), the number of repeats can be deduced from the length of the amplicon. This information is currently almost exclusively obtained by capillary electrophoresis, a well-known DNA separation technique. Size separation of the amplicons is achieved by applying a high electric potential across a gel-filled capillary through which the amplicons move. Differences in electrophoretic mobility will result in faster migration of shorter amplicons. These fluorescently labeled DNA-fragments are detected by laser-induced fluorescence (LIF) [2].

Although efforts are made to create a portable variant of the tools used for CE (e.g. the RapidHIT system) [5] or by miniaturizing this technique on a chip (e.g. glass) [6], CE still requires rather bulky equipment. Interest in implementing Lab-on-a-Chip (LoC) in the field of forensic DNA genotyping is indeed rapidly growing, owing to a myriad of benefits, such as shorter analysis times and decreased reagent consumption, but also a high degree of parallelization and flexibility. Furthermore, production cost of the equipment would decrease, and user friendliness would dramatically increase. Another major advantage, especially in the field of forensics, is the reduced risk of contamination. LoCs show a high level of integration, combining several functionalities into a single device and eliminating the need for transporting a sample from one device to another. By making DNA analysis portable, transport to an accredited laboratory becomes redundant, again minimizing the risk for contamination and enabling a faster turnaround time. Quick results are crucial for those entrusted with the task of solving crimes, as the first hours of such an investigation are commonly called the "golden hours".

A lot of effort has already been done to develop new assays, mostly hybridization based. This implicates that synthetically manufactured oligonucleotides, mostly fluorescently labeled, are used for STR genotyping. These techniques show some benefits in comparison to CE, for example no high voltages should be applied. Another issue related to CE, namely the detection of PCR-artefacts, is omitted, as these artefacts do not hybridize with the probes added to the system.

A well-known example of a hybridization-based method for STR genotyping is the use of HyBeacon^{™} probes for melting curve analysis, as described in EP3011053A2 [7]. In this technique, only one fluorescently labeled probe per locus is used and the number of repeats is deduced from the melting temperature of the said probe [8]. Drawbacks to this method are multiple: i) the need for a second oligonucleotide functioning as a blocker, ii) the presence of multiple internally positioned fluorophores, thereby increasing the cost of probes and iii) probe design restrictions, making it impossible to design a system capable of genotyping all the loci needed for a complete DNA profile.

Most alternative hybridization-based techniques rely on the use of one or more fluorophores combined with a quencher moiety, thereby increasing the cost of the probe and the complexity of the system. Examples are TaqMan probes (U.S. Pat. Nos. 5,210,015) [9] and Molecular Beacons (US Pat. No. 6150097A) [10]. Other systems use multiple fluorophores, again increasing the cost of the probe and the complexity of the system. Examples of these systems are Scorpion probes [11] and ECHO probes [12]. It should be noted that most of these probes are not designed for STR-genotyping.

The principle of Fluorescent Resonant Energy Transfer (FRET) between a donor and an acceptor moiety is often exploited in DNA-probe based methods. In most cases, both moieties are attached to (one of) the oligonucleotides from which the system exists. Halpern et al. developed a melt curve genotyping assay relying on FRET, by combining an intercalating dye and a fluorescently labeled oligonucleotide. Upon melting, FRET between the intercalating dye and the oligonucleotide disappears and a decrease in fluorescent signal can be observed. The actual STR-allele calling in the said system is based on Tm detection, as mismatch results in a lower Tm compared to a perfect match [13], US12/276849 ([14]).

Although, in the event of a mismatch, the reporter region of the probe is not expected to hybridize with the amplicon, implicating the absence of intercalating dye in this region, rather high and sharp melting curves are observed for these mismatch probes, albeit at a lower temperature. This is probably due to the presence of intercalating dye in the rest of the duplex, and the presence of intercalating dye in the vicinity of the single stranded DNA. It would be beneficial if mismatch melt curves should not only occur at a lower temperature, but also have another shape (e.g. lower peak height, higher peak width). This would enable correct STR-genotyping despite the presence of a SNP located in the flanking regions of the probe. A mismatch at the SNP position combined with a match at the STR-region would result in melt curves with a peak shape similar to normal match probes, but at a lower temperature.

The use of an intercalating dye is indissolubly linked to some disadvantages, e.g. the inhibition of PCR. As forensic samples often contain only very small amounts of DNA, PCR should preferably take place in ideal circumstances. PCR inhibition is the reason why intercalating dyes are often added in sub-saturating concentrations, thereby increasing the risk of dye jumping. This phenomenon relates to intercalating dye which is released from a duplex upon melting but gets incorporated in another duplex that has not yet melted, resulting in broadening of the melting peaks. Furthermore, intercalating dyes bind preferentially to regions containing a lot of guanine and cytosine (so-called 'GC-rich regions'). This potentially results in a more pronounced signal for these loci. At last, the concentration of intercalating dye influences the melting temperature of a duplex quite pronounced, thereby introducing a new source of variability of the melting temperature, which is by all means detrimental when performing a melt curve genotyping assay. [15] Apart from these technical limitations, one should take in consideration the risks to human health related to the use of intercalating dyes.

All these examples indicate the need for a hybridization-based genotyping assay which is more simple and robust on the one hand, enabling integration in a portable device with ease, and results in more information than solely a melting temperature on the other hand. In our view, the most simple probe possible contains only one fluorophore, and generates a signal solely based on interaction with the sample, without the use of any other modifications (e.g. a quencher), other molecules (e.g. intercalating dyes) or even other probes (e.g. a blocker).

Probes relating solely on the quenching properties of naturally occurring nucleotides have already demonstrated their utility in other applications, e.g. species identification, qPCR and SNP genotyping, as described by Wittwer *et al.* in EP2927238A1. [16] In the said document, so-called Q-probes for SNP genotyping are described. These probes are designed in such a way that the fluorescently labeled nucleotide always hybridizes to the target sequence, regardless the fact whether or not the probe matches the amplicon at the SNP position. By doing so, quenching will appear upon hybridization, irrespective the genotype of the examined sample. Genotyping is performed by standard melting curve analysis, as a mismatch between probe and sample will lower the melting temperature of the duplex. The quenching effect of the sample on the fluorophore is not associated to the fact whether the probe and amplicon match perfectly or not, and variant calling is based on one factor, the melting temperature. This has proven to be sufficient informative for SNP calling, whereas for STR-genotyping, a rather broad range of alleles are possible for every investigated locus, making the exploitation of probes as described by Wittwer *et al.* or the above described HyBeacon probes non-useful. STR-loci have other structural properties as compared to SNP-loci, as the possible alleles differ in length rather than only in sequence. Therefore, for every locus, an array of probes of different length should be developed along with a method to assess whether or not a probe is completely complementary to the sample, which is in sharp contrast to the probes described by Wittwer *et al.* where only one probe is designed for a certain locus.

There is thus still a need to design simple probes which are useful for STR-genotyping, as some structural elements (e.g. an anchor region and a sensor region) are indispensable.

### Brief description of figures

**Figure 1** : STR genotyping example for the D16S539 locus. Three duplexes of probe (upper) and STR-amplicon (lower) are shown. Each amplicon contains twelve STR repeats, while the depicted probes contain eleven, twelve or thirteen repeats. The probes consist, from 5' to 3', of a FL1 region (black), a specific number of repeats (dark grey) and the Fl2 region containing one or more fluorophores (light grey). When the amplicon contains the exact equal number of repeats as the probe, the FL2 region will occur in a hybridized state, resulting in quenching of the fluorophore(s).
**Figure 2****:** Typical example of a melting experiment. Before melting, fluorescence decreases in a linear fashion, and probes are hybridized to an amplicon, with quenching of the fluorophore as a consequence. Upon melting, a sudden increase of fluorescence is observed due to the loss of the quenching effect of guanine. After melting, fluorescence again decreases in a linear fashion, and probes are single stranded.

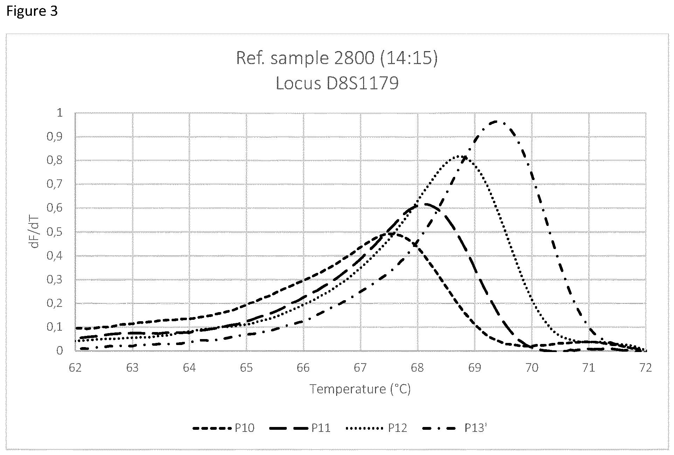

**Figure 3****:** Melting curves of mismatch probes for the D8S1179 locus.
**Figure 4****:** Temperature profile for the D16S539 experiment.
**Figure 5****:** Melting peaks of the probes designed for the D16S539 locus after hybridization with a heterozygous sample (9:12).
**Figure 6****:** Melting peaks of the probes designed for the D16S539 locus after hybridization with a homozygous sample (9:9).
**Figure 7****:** Melting peaks of the probes designed for the D16S539 locus after hybridization with a heterozygous sample (11:13).

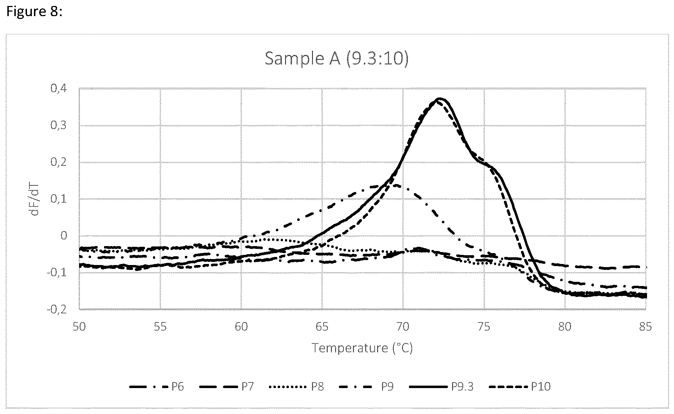

**Figure 8****:** Melting peaks of the probes designed for the TH01 locus after hybridization with a heterozygous sample (9.3:10).

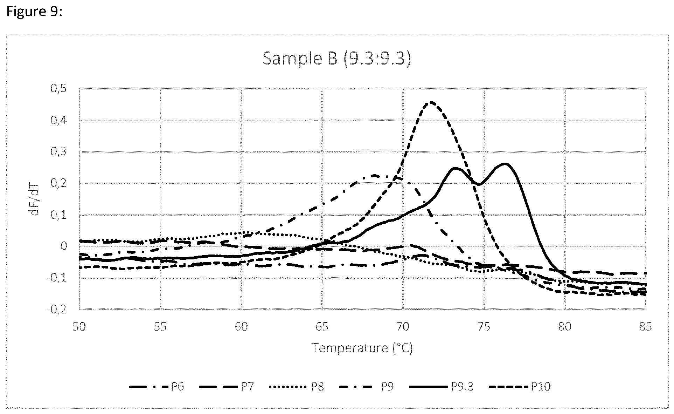

**Figure 9****:** Melting peaks of the probes designed for the TH01 locus after hybridization with a homozygous sample (9.3:9.3).

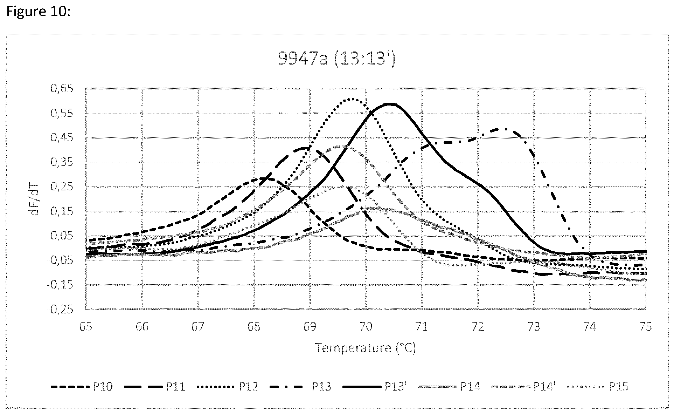

**Figure 10****:** Melting peaks of the probes designed for the D8S1179 locus after hybridization with a heterozygous sample (13:13').

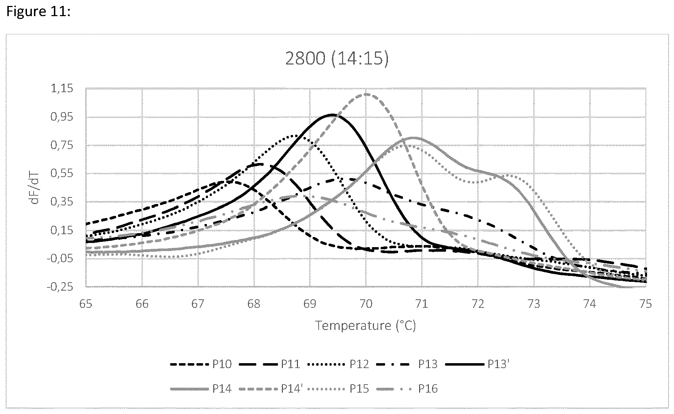

**Figure 11****:** Melting peaks of the probes designed for the D8S1179 locus after hybridization with a heterozygous sample (14:15).

### Summary of the invention

The present invention relates to a plurality of probes representing the allelic variability of a certain short tandem repeat locus within a population, wherein each probe consists, from 5' to 3' or from 3' to 5', of: 1) a first flanking region which comprises nucleotides and which anneals with a region directly next to the specific DNA sequence of interest and which ensures proper annealing of the sample and the probe and which therefore contains more nucleotides than the second flanking region, 2) a specific DNA sequence of interest which comprises at least 1 short tandem repeat and which anneals with the short tandem repeat region within the sample, and 3) a second flanking region which comprises at least 1 nucleotide and which contains at least one fluorophore and wherein said fluorophore is attached to a residue of said second flanking region in the position directly complementary to a specific nucleotide capable of quenching the said fluorophore in an efficient way of said sample, or linked to a nucleotide adjacent -either upstream or downstream- to said position or linked to a nucleotide 2 positions away -either upstream or downstream- of said position so that it is brought into close proximity of one or more specific nucleotides capable of quenching the said fluorophore in an efficient way of said sample upon hybridization of said second flanking region with the sample.

The present invention further relates to a plurality of probes as described above wherein said nucleotides are nucleic acid analogues, e.g. LNAs.

More specifically, the present invention relates to a a plurality of probes as described above wherein said fluorophore is chosen from the list comprising fluorescein (FAM), hexachlorofluorescein (HEX), tetrachloro-6-carboxyfluorescein (TET), 2,7-dimethoxy-4,5-dichloro-6- carboxyfluorescein (JOE) or 6-carboxytetramethylrhodamine (TAMRA). The specific nucleotide capable of quenching one of the above listed fluorophores is guanine.

More specifically, the present invention relates to a a plurality of probes as described above wherein said fluorophore is attached to a cytosine residue of said second flanking region.

The present invention also relates to a plurality of probes as described above which is immobilized on a support.

The present invention also relates to a method to genotype short tandem repeats within a sample comprising the steps of:
- providing a sample comprising DNA,
- amplifying DNA within said sample which comprises a specific DNA sequence of interest in order to obtain amplified DNA sequences,
- adding a plurality of probes as described above to said amplified DNA sequences to obtain duplexes of single stranded DNA sequences annealed to said probe, and
- denaturing said duplexes followed by slowly cooling said denatured duplexes while continually measuring fluorescence of the fluorophore of said probe, or, slowly heating said duplexes while continually measuring fluorescence of the fluorophore of said probe, wherein the decrease of fluorescence intensity or the increase in fluorescence intensity, respectively, provides information on whether or not a specific, completely complementary short tandem repeat is present in said sample.

The present invention further relates to a method to genotype as described above wherein said amplification within said sample is undertaken by an asymmetric PCR in order to obtain amplified, single stranded DNA sequences.

The present invention further relates to a method to genotype as described above wherein said amplification within said sample is undertaken by a symmetric PCR using biotin-labeled primers or a subsequent lambda exonuclease digestion in order to obtain amplified, single stranded DNA sequences.

The present invention also relates to a method as described above wherein said probes are added in solution, or are immobilized onto a support.

### Description of the invention

An aspect of this invention relates to probes whose functioning rely on the naturally occurring quenching properties of specific nucleotides on certain fluorophores. The most common example is the fluorescein-quenching effect of guanine [17, 18]. Another example is the quenching of pyrenebutyrate by thymidine nucleotides.

A probe is herein defined as a synthetically manufactured oligonucleotide wherein some nucleotides might be modified. Examples of modifications are e.g. the presence of a fluorescent moiety, molecules for attachment purposes, etc. Probes are generally designed in such a way that they will interact with the investigated molecule, and the response of the probe upon this interaction will be observed and used in order to obtain information of the said investigated molecule.

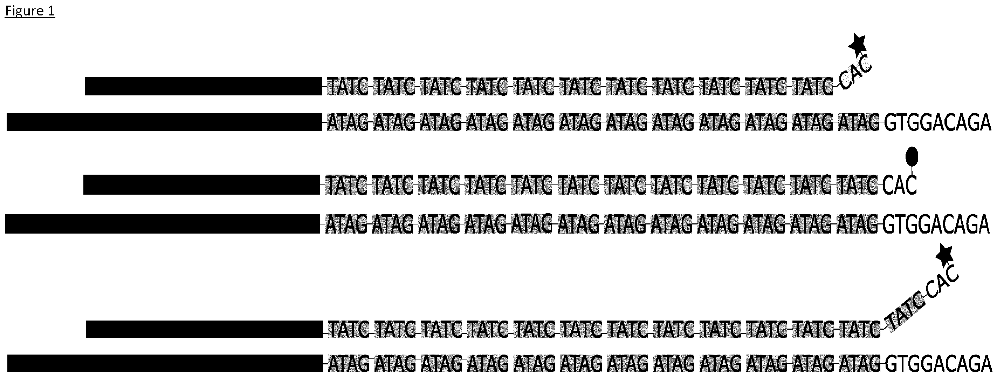

STR genotyping probes described in this invention consist of three different regions, as shown in figure 1: flanking region 1 (FL1), the specific STR-region, and flanking region 2 (FL2).
- FL1 is the region directly next to the specific DNA sequence and acts as an anchor, ensuring proper annealing of the sample and the probe, preventing slippage. This implicates that FL1 should be significantly longer than FL2, a requirement which is also discussed in literature on other STR-genotyping probes [13]. If FL1 would be as long as or even shorter than FL2, FL1 would be single stranded in the case of mismatch, and FL2 would hybridize with the sample, thereby resulting in a signal comparable to the signal generated by a matching duplex. An extra functionality can be added at the terminus of FL1 for attachment purposes.
- The STR-region is the polymorphous part which differs between probes for a certain locus. A probe is designed for every possible allele of the examined locus.
- FL2 is substantially shorter than FL1 and is terminally labeled with a fluorophore, e.g. FAM. This labeling can be either 5' or 3' terminal. FL2 acts as a sensor, and gives an indication on the degree of complementarity between the probe and the sample.

The probe is designed in such a way that, upon hybridization with a complementary amplicon, the fluorophore is brought into proximity of one or more nucleotides capable of quenching said fluorophore. In a more specific embodiment of this invention, said fluorophore is FAM, which is quenched by the presence of guanine residues. These guanine residues also exert a quenching effect on other fluorophores, like HEX, TET, JOE and TAMRA [19]. Those skilled in the art will recognize that this is a non-limitative list. It should be noted that other combinations of fluorophores and nucleotides are also applicable for this purpose. In order to achieve efficient quenching of the FAM fluorophore, said fluorophore is linked to a nucleotide (mostly cytosine) in the position directly complementary to the guanine residue, or linked to a nucleotide adjacent to the said position (either upstream or downstream) or linked to a nucleotide 2 positions away from the said position (either upstream or downstream).

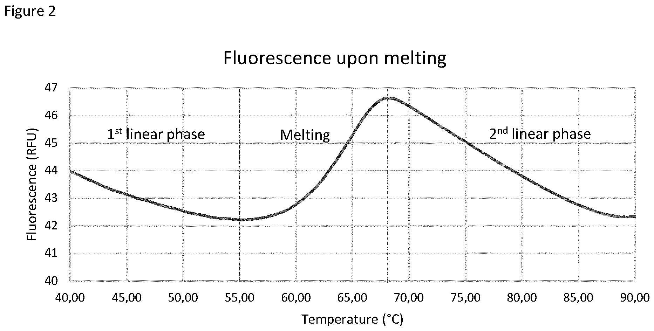

In the herein described method, an array of probes is designed, representing all possible alleles of a certain STR-locus. The difference between full complementarity and partial complementarity of an amplified sample and the probe can be assessed by measuring the fluorescence intensity of the fluorophore attached to the probe upon hybridization or melting. Resulting fluorescence graphs as a function of time can be divided in 3 parts (see figure 2): a linear part, during which the fluorescence decreases (temperature-dependent phenomenon) and (most of) the probes are hybridized to an amplicon, with quenching of the fluorophore as a consequence, the melting part during which the fluorescence increases, and a second linear phase where the probes are single stranded. Calculating the first derivative of these graphs as a function of temperature provides melting peaks used for interpretation of the data. After amplification, probe and amplicons are denatured by heating and thereafter cooled down slowly in a controlled fashion, ensuring correct hybridization by avoiding slippage of the probe. The probe-amplicon duplexes are thereafter melted, while fluorescence is measured constantly. Upon melting, the distance between the fluorophore and the quenching guanine residue will increase and the fluorescence intensity will increase. This increase in fluorescence intensity occurs at a higher temperature and is more pronounced when the probe and the amplicon present in the PCR product share the same repeat number, as compared to a mismatch combination of probe and amplicon. Still some dequenching can be observed upon melting in the event of a mismatch situation, due to the formation of hetero-duplexes. These duplexes contain a mismatch in the repeat region, resulting in the formation of a bulged loop. However, the melting temperature of these duplexes is lower as compared to full complementarity, and the hybridization efficiency is pronouncedly lower: for most probes, the sensor region will remain single stranded.

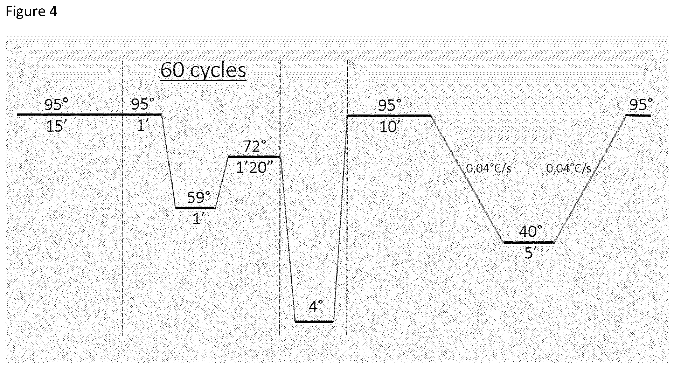

The herein described probes provide information on the degree of complementarity between probe and sample. Besides deducing whether or not probe and sample have the same number of repeats, information on the number of repeats differing between sample and probe can be obtained in the case of a mismatch. The bigger the difference in repeat number, the lower the obtained signal will be. An example is given in figure 4, for the D8S1179 locus. The melting curves of 4 probes are displayed, after incubation with reference sample 2800, which has alleles 14 and 15. All the shown melting curves originate from mismatch probes. It can clearly be seen that probe 13 shows the most intense signal, and probe 10 the less intense. Intensity of the signal can in this example be defined by the height of the melting peak and the melting temperature.

A unique feature of this invention is the considerable amount of information that can be retrieved from multiple parameters of these melting curves (Tm, peak shape,...). Melting curves result in an indication of the degree of complementarity, whereas most systems solely give a binary answer (match or mismatch). The latter systems only look at one parameter, e.g. the melting temperature or the fluorescence intensity. The unique positioning of the fluorophore, in combination with the other structural elements of the probes, makes these STR-probes highly informative. The fluorophore is positioned in the second flanking region, thereby acting as a sensor: when the probe matches the sample, FL2 will hybridize to the said sample. Whereas, when the probe does not match the sample, FL2 mainly remains single stranded, or melts at a lower temperature, and dequenching upon melting will occur less sudden. The bigger the distance between the fluorophore and the amplicon with quenching moieties, the less intense the signal will be. Therefore, to our knowledge, the STR genotyping probe discussed herein is the most elementary and informative STR genotyping probe described yet, as both the melting temperature and the fluorescence intensity are informative.

This obtained information can eventually be analyzed in an automated way by means of artificial intelligence. Similar algorithms for high resolution melting analysis have already been described. A custom algorithm for allele calling could be developed in the future, based on a high amount of data. For this purpose, the said algorithm should be trained for calling the correct alleles, based on the curves of samples with known alleles.

An important aspect for hybridization-based genotyping methods is the requirement of an excess of the amplicon complementary to the probe. If this requirement would not be met, both amplicon strands would hybridize preferentially to each other, leaving the probe single stranded. An excess of one amplicon strand can be obtained by adapting the amplification step. After sample preparation, an amplification step should be performed in order to amplify the STR-loci. This is typically done by means of the polymerase chain reaction (PCR), a technique well known to those skilled in the art. The region(s) being amplified are determined by the primers used. These are short oligonucleotides complementary to a sequence in the genome of the examined species. The DNA polymerase will initiate amplification at the 3' terminus of the primer.

In symmetric PCR, both primers are added in equal concentrations, resulting in double stranded amplicons. When asymmetric PCR is performed, one primer is added in excess. In the first cycli, both primers are present and PCR occurs symmetric. At a certain point, one primer will be depleted, resulting in the amplification of only 1 of the 2 strands. From this point on, amplification will not occur exponentially but linear.

Asymmetric PCR is not the only way to obtain an excess of one specific strand. After performing symmetric PCR where one of both primers is labelled with biotin, the strands in which this primer is incorporated can be captured by means of streptavidin beads. Another option is the use of the lambda exonuclease enzyme, which selectively degrades phosphorylated DNA-strands. This modification can be introduced in 1 of the 2 primers. [20]

The probe as described above can also contain nucleic acid analogues, e.g. LNAs. The former are non-naturally occurring components which resemble structurally to the naturally occurring nucleic acids. Among many other examples are nucleic acids with a modified base, or a modification in the sugar component.

### Examples

### 1. EXAMPLE 1: STR- genotyping buccal swabs (D16S539 locus)

Three buccal swabs were immersed in a volume of 200 µL sterile HPLC-water. After a vortex-step of 30", the swab was removed and the water was used as input for PCR. Singleplex asymmetric PCR was performed with 30 µL of input sample. Primer concentrations were 0.1 µM forward primer and 1.5 µM reverse primer. The volume of the PCR mixture was 50 µL, containing MgCl²⁺ at a concentration of 0.5 mM, dNTPs at 200µM each, 1X Qiagen PCR buffer and 1.3U HotStarTaq enzyme. Activation of the polymerase was done by heating the PCR mix at 95°C for 15 minutes followed by 60 cycles of 95°C for 1 minute, 59°C for 1 minute and 72°C for 80 seconds. Primer sequences can be found in table 1.

After asymmetric PCR, aliquots of 8.5 µL amplified product were divided in a 96-Well plate. To each separate well, 1.5 µL of one particular probe was added at a starting concentration of 1 µM. These mixtures were denatured for 10 minutes at 95°C, followed by slowly cooling at a ramp rate of 0.04 °C/s while fluorescence was continuously measured using a LightCycler (Roche). The same was done upon slowly heating, during this process duplexes will melt. Probe sequences can be found in table 1.

**Table 1: Sequence of oligonucleotides used for the D16S539 experiment. 'n' denotes the number of repeats and varied between 9 and 13.**

| **Name** | **Sequence** | **SEQ ID N°** |
|---|---|---|
| D16S539 Forward primer | 5' GGGGGTCTAAGAGCTTGTAAAAAG | 1 |
| D16S539 Reverse primer | 5' GTTTGTGTGTGCATCTGTAAGCATGTATC | 2 |
| D16S539 Probe | 5' GTTTTGTCTTTCAATGA(TATC)ₙCAC/36-FAM/ | 3 (n=9), 4 (n=10), 5 (n=11), 6 (n=12), 7 (n=13) |

The first derivative of the melting curve is calculated, resulting in melting peaks. Differences in melting temperature due to a difference in length of the probe can be examined in this way. All examined samples were also genotyped with conventional CE-analysis as a reference.

### 2. EXAMPLE 2: STR- genotyping buccal swabs (TH01 locus)

In order to assess the ability of this system to detect rather subtle differences in amplicon length, caused by partial repeats, a melting curve experiment using the probes designed for the TH01 locus was carried out. A quite common allele for this locus is the 9.3 allele, which is characterized by the presence of 10 repeats (CATT), of which the 4^{th} repeat has a T-deletion. As a consequence, alleles 9.3 and 10 only differ one nucleotide in length, which has even for CE proven to be a challenge. Two buccal swabs were extracted, amplified and analyzed in the same way as the experiment for the D16S539 locus. As opposed to the amplification for the latter locus, forward primer was added in a concentration of 0,1 µM and the reverse primer was added in a concentration of 1,5 µM. Sequence of primers and probes used can be found in table 2. Sample A has alleles 9.3 and 10; sample B is homozygous (9.3:9.3).

**Table 2: Sequence of oligonucleotides used for the TH01 experiment. 'n' denotes the number of repeats and varied between 6 and 10.**

| **Name** | **Sequence** | **SEQ ID N°** |
|---|---|---|
| TH01 Forward primer | 5'GTGATTCCCATTGGCCTGTTC | 8 |
| TH01 Reverse primer | 5'GTTTGTGTGTGCATCTGTAAGCATGTATC | 9 |
| TH01 Probe | 5'/56-FAM/CCT(CATT)n CACCATGGAGTCTGTGTTCCCTGTG | 10 (n=6), 11 (n=7), 12 (n =8), 13 (n=9), 14 (n=10) |
| TH01 Probe 9.3 | 5’/56- FAM/CCT(CATT)3CAT(CATT)6CACCATGGAGTCTGT GTTCCCTGTG | 15 |

### 3. EXAMPLE 3: STR-genotyping reference samples (D8S1179 locus)

In order to assess the ability of this system to detect iso-alleles, caused by a SNP in a repeat, a melting curve experiment using the probes designed for the D8S1179 locus was carried out. Reference sample 9947a is homozygous for locus D8S1179 (13:13), but is genotyped by means of massive parallel sequencing as 13:13'. Sequences corresponding to alleles 13 and 13' can be found in table 3. Reference sample 2800 is heterozygous for locus D8S1179 (14:15). Both reference samples are amplified and analyzed in the same way as the experiment for the D16S539 locus. As opposed to the amplification for the latter locus, forward primer was added in a concentration of 0,1 µM and the reverse primer was added in a concentration of 1,5 µM. Sequence of primers and probes used can be found in table 3.

**Table 3: Sequence of oligonucleotides used for the D8S1179 experiment.**

| **Name** | **Sequence** | **SEQ ID N°** |
|---|---|---|
| D8S1179 Forward primer | 5' ATTGCAACTTATATGTATTTTTGTATTTCATG | 16 |
| D8S1179 Reverse | 5' ACCAAATTGTGTTCATGAGTATAGTTTC | 17 |
| primer | | |
| D8S1179 Probe 10 | 5’ TTGTATTTCATGTGTACATTCGTA(TCTA)10TTCCC/3 6-FAM/ | 18 |
| D8S1179 Probe 11 | 5’ TTGTATTTCATGTGTACATTCGTA(TCTA)11TTCCC/36 -FAM/ | 19 |
| D8S1179 Probe 12 | 5' TTGTATTTCATGTGTACATTCGTA(TCTA)12TTCCC/36 -FAM/ | 20 |
| D8S1179 Probe 13 | 5’ TTGTATTTCATGTGTACATTCGTA(TCTA)(TCTG)(TCT A)11TTCCC/36-FAM/ | 21 |
| D8S1179 Probe 13' | 5' TTGTATTTCATGTGTACATTCGTA(TCTA)13TTCCC/36 -FAM/ | 22 |
| D8S1179 Probe 14 | 5’ TTGTATTTCATGTGTACATTCGTA(TCTA)(TCTG)(TCT A)12TTCCC/36-FAM/ | 23 |
| | | |

| **Name** | **Sequence** | **SEQ ID N°** |
|---|---|---|
| D8S1179 Probe 14' | 5' TTGTATTTCATGTGTACATTCGTA(TCTA)14TTCCC/36 -FAM/ | 24 |
| D8S1179 Probe 15 | 5' TTGTATTTCATGTGTACATTCGTA(TCTA)(TCTG) (TCTA)13TTCCC/36-FAM/ | 25 |
| D8S1179 Probe 16 | 5’ TTGTATTTCATGTGTACATTCGTA(TCTA)(TCTG) (TCTA)14TTCCC/36-FAM/ | 26 |

### Results

### 1. EXAMPLE 1: STR- genotyping buccal swabs (D16S5339 locus)

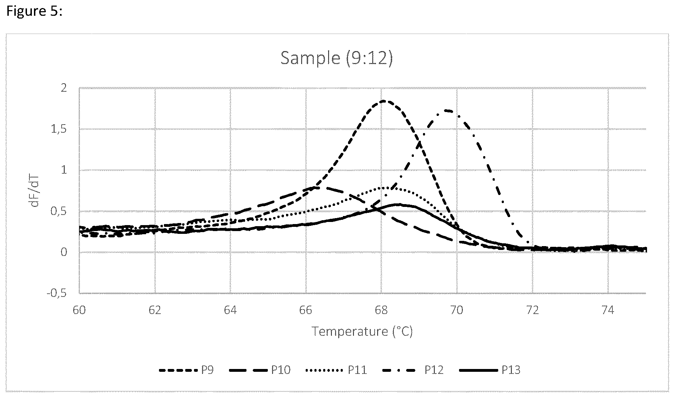

The first derivative of the obtained melting curves was calculated, the resulting melting peaks are shown in figures 5-7. Figure 5 displays the melting peaks obtained from sample 7 with alleles 9 and 12, where the allele 12 probe (P12) melts at a higher temperature as compared to the allele 9 probe (P9).

As can be seen in figure 5, every probe shows some kind of melting peak. Nevertheless, P9 and P12 display a much higher peak height and a more narrow peak width. P11 shows the most intense melting peak of the mismatch probes, which is sequacious as this is a neighboring allele of the matching probe 12. Nevertheless, the difference of Tm between P11 and P12 is too big, indicating non-specific annealing of P11.

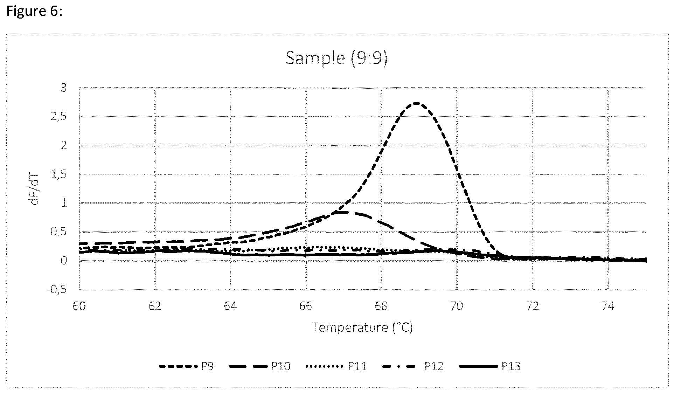

Figure 6 displays the melting curves of a homozygous sample (alleles 9:9). Melting peaks of matching probes are more pronounced as compared to heterozygous samples.

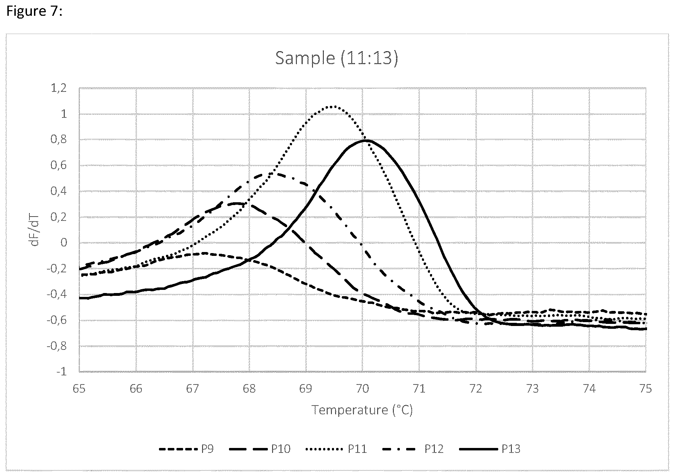

Figure 7 displays the melting curves of a heterozygous sample (alleles 11 and 13). The probe with 12 repeats is a neighboring probe of both matching probes but still a clear distinction can be made between match and mismatch.

Summarizing, sufficient information for genotyping can be deduced from hybridization or melting experiments. It should be noted that, when a melting experiment is carried out, a slow hybridization process should precede in order to guarantee specific annealing of the probes.

### 2. EXAMPLE 2: STR- genotyping buccal swabs (TH01 locus)

It should be noted that, for most of the examined loci, matching probes display 2 peaks, whereas mismatch probes only 1. This is most probably due to the presence of another allele (heterozygous samples), stutter peaks and aspecific PCR-products. Evaluation of these melting curves are by consequence less complicated. However, for the D16S539 locus, matching alleles show only one peak, which is most probably related to the shorter FL1 of these probes.

For sample A, 2 probes display melting peaks at a higher temperature, and in addition these peaks are characterized by a so-called "shoulder", which is in fact a second peak as discussed above. The 2 probes correspond to the correct alleles. For the homozygous sample B, only one probe shows a melting peak at a higher temperature, corresponding to allele 9.3. Although probe 10 displays a higher melting peak, it occurs at a lower temperature, and a shoulder peak is absent. Thereby, it can be concluded that allele 10 is not present in the examined sample. Besides that, one can conclude that the described probes and system is able to distinguish between allele 9.3 and 10.

### 3. EXAMPLE 3: STR-genotyping reference samples (D8S1179 locus)

For sample 9947a, both probe 13 and 13' display melting peaks at a higher temperature. Besides that, both melting peaks show so-called shoulders, similar to the TH01 probes. Therefore, it can be concluded that the complementary amplicons for both probes 13 and 13' are present in sample 9947a. For sample 2800, probe 14 and 15 show melting peaks at a higher temperature, with shoulders. Probe 14' however does not show a shoulder, and occurs at a lower temperature. The high peak-height can be explained by the presence of both alleles 14 (which has the same length) and 15 (which is a close neighbor). It can be concluded that this method is capable of distinguishing iso-alleles, thereby being more informative than capillary electrophoresis.

### References

1. Westen, A.A., et al., Tri-allelic SNP markers enable analysis of mixed and degraded DNA samples. Forensic Science International: Genetics, 2009. 3(4): p. 233-241.
2. Butler, J.M., Forensic DNA typing: biology, technology, and genetics of STR markers. 2005: Elsevier.
3. Schneider, P.M., Expansion of the Europian standard set of DNA database loci-the current situation. Profiles in DNA, 2009. 12: p. 6-7.
4. Hares, D.R., Selection and implementation of expanded CODIS core loci in the United States. Forensic Science International: Genetics, 2015. 17: p. 33-34.
5. Hennessy, L.K., et al., Developmental validation studies on the RapidHIT™ human DNA identification system. Forensic Science International: Genetics Supplement Series, 2013. 4(1): p. e7-e8.
6. Bruijns, B., et al., Microfluidic devices for forensic DNA analysis: A review. Biosensors, 2016. 6(3): p. 41.
7. French, D., et al., Fluorophore-based oligonucleotide probes with a universal element. 2016*,* EP3011053A2.
8. Gale, N., et al., Rapid typing of STRs in the human genome by HyBeacon® melting. Organic & biomolecular chemistry, 2008. 6(24): p. 4553-4559.
9. Gelfand, D.H., et al., Homogeneous assay system using the nuclease activity of a nucleic acid polymerase. 1993, US Patent No. 5,210,015.
10. Tyagi, S. and F.R. Kramer, Nucleic acid detection probes having non-FRET fluorescence quenching and kits and assays including such probes. 2000, US6150097.
11. Thelwell, N., et al., Mode of action and application of Scorpion primers to mutation detection. Nucleic acids research, 2000. 28(19): p. 3752-3761.
12. Okamoto, A., ECHO probes: a concept of fluorescence control for practical nucleic acid sensing. Chemical Society Reviews, 2011. 40(12): p. 5815-5828.
13. Halpern, M.D. and J. Ballantyne, An STR melt curve genotyping assay for forensic analysis employing an intercalating dye probe FRET. Journal of forensic sciences, 2011. 56(1): p. 36-45.
14. Halpern, M. and P.M. Ellis, Dye probe fluorescence resonance energy transfer genotyping. 2010, US12/276849.
15. Reed, G.H., J.O. Kent, and C.T. Wittwer, High-resolution DNA melting analysis for simple and efficient molecular diagnostics. 2007.
16. Wittwer, C.T., et al., Single-labeled oligonucleotide probes for homogeneous nucleic acid sequence analysis. 2003, Google Patents.
17. Cooper, J.P. and P.J. Hagerman, Analysis of fluorescence energy transfer in duplex and branched DNA molecules. Biochemistry, 1990. 29(39): p. 9261-9268.
18. Lee, S.P., et al., A fluorometric assay for DNA cleavage reactions characterized with BamHl restriction endonuclease. Analytical biochemistry, 1994. 220(2): p. 377-383.
19. Mao, H., et al., The mechanism and regularity of quenching the effect of bases on fluorophores: the base-quenched probe method. 2018. 143(14): p. 3292-3301.
20. Marimuthu, C., et al., Single-stranded DNA (ssDNA) production in DNA aptamer generation. Analyst, 2012. 137(6): p. 1307-1315.

## Claims

1. A plurality of probes representing the allelic variability of a certain short tandem repeat locus within a population consisting, wherein each probe consists, from 5' to 3' or from 3' to 5', of: 1) a first flanking region which comprises nucleotides and which anneals with a region directly next to the specific DNA sequence of interest and which contains more nucleotides than the second flanking region, 2) a specific DNA sequence of interest which comprises at least 1 short tandem repeat and which anneals with the short tandem repeat region within the sample, and 3) a second flanking region which comprises at least 1 nucleotide and which contains at least one fluorophore and wherein said fluorophore is attached to a residue of said second flanking region in the position directly complementary to a specific nucleotide capable of quenching the said fluorophore in an efficient way of said sample, upon hybridization of said second flanking region within the sample, and wherein said specific nucleotide capable of quenching the said fluorophore in an efficient way is guanosine, and wherein said fluorophore is chosen from the list comprising fluorescein (FAM), hexachlorofluorescein (HEX), tetrachloro-6-carboxyfluorescein (TET), 2,7-dimethoxy-4,5-dichloro-6- carboxyfluorescein (JOE) or 6-carboxytetramethylrhodamine (TAMRA)

2. A plurality of probes according to claim 1 wherein said fluorophore is attached to a cytosine residue of said second flanking region.

3. A plurality of probes according to claims 1 or 2 wherein said nucleotides are nucleic acid analogues.

4. A plurality of probes according to any of claims 1-3 which is immobilized on a support.

5. A method to genotype short tandem repeats within a sample comprising the steps of:
- providing a sample comprising DNA,
- amplifying DNA within said sample which comprises a specific DNA sequence of interest in order to obtain amplified DNA sequences,
- adding a plurality of probes according to claims 1-4 to said amplified DNA sequences to obtain duplexes of single stranded DNA sequences annealed to said probe, and
- denaturing said duplexes followed by slowly cooling said denatured duplexes while continually measuring fluorescence of the fluorophore of said probe, or, slowly heating said duplexes while continually measuring fluorescence of the fluorophore of said probe, wherein the decrease of fluorescence intensity or the increase in fluorescence intensity, respectively, provides information on whether or not a specific, completely complementary short tandem repeat is present in said sample.

6. A method to genotype according to claim 5 wherein said amplifying DNA within said sample is undertaken by an asymmetric PCR in order to obtain amplified, single stranded DNA sequences.

7. A method to genotype according to claim 5 wherein said amplifying DNA within said sample is undertaken by a symmetric PCR using biotin-labeled primers or a subsequent lambda exonuclease digestion in order to obtain amplified, single stranded DNA sequences.

8. A method according to claims 5-7 wherein said probes are added in solution or immobilized on a support.

## Patentansprüche

1. Mehrere Sonden, repräsentierend die allelische Variabilität eines bestimmten Short Tandem Repeat-Locus in einer bestehenden Population, wobei die Sonden jeweils von 5' nach 3' oder von 3' nach 5' aus 1) einer ersten flankierenden Region, die Nukleotide umfasst und die sich per Annealing an eine Region direkt neben der spezifischen DNA-Sequenz von Interesse anlagert und die mehr Nukleotide als die zweite flankierende Region enthält, 2) einer spezifischen DNA-Sequenz von Interesse, die mindestens 1 Short Tandem Repeat umfasst und die sich per Annealing an die Short-Tandem-Repeat-Region in der Probe anlagert, und 3) einer zweiten flankierenden Region, die mindestens 1 Nukleotid umfasst und die wenigstens einen Fluorophor enthält, und wobei der Fluorophor an einen Rest der zweiten flankierenden Region in der Position gebunden ist, die zu einem spezifischen Nukleotid der Probe direkt komplementär ist, das den Fluorophor nach Hybridisierung der zweiten flankierenden Region innerhalb der Probe wirksam quenchen kann, und wobei es sich bei dem spezifischen Nukleotid, das den Fluorophor wirksam quenchen kann, um Guanosin handelt und wobei der Fluorophor aus der Liste, die Fluorescein (FAM), Hexachlorfluorescein (HEX), Tetrachlor-6-carboxyfluorescein (TET), 2,7-Dimethoxy-4,5-dichlor-6-carboxyfluorescein (JOE) oder 6-Carboxytetramethylrhodamin (TAMRA) umfasst, gewählt ist.

2. Mehrere Sonden nach Anspruch 1, wobei der Fluorophor an einen Cytosinrest der zweiten flankierenden Region gebunden ist.

3. Mehrere Sonden nach Anspruch 1 oder 2, wobei es sich bei den Nukleotiden um Nukleinsäureanaloge handelt.

4. Mehrere Sonden nach einem der Ansprüche 1 bis 3, die an einem Träger immobilisiert sind.

5. Verfahren zur Genotypisierung von Short Tandem Repeats in einer Probe, umfassend die Schritte:
- Bereitstellen einer DNA umfassenden Probe,
- Amplifizieren von DNA in der Probe, die eine spezifische DNA-Sequenz von Interesse umfasst, um amplifizierte DNA-Sequenzen zu erhalten,
- Addieren mehrerer Sonden nach Anspruch 1 bis 4 an die amplifizierten DNA-Sequenzen unter Erhalt von Duplexen einzelsträngiger DNA-Sequenzen, die per Annealing an die Sonde angelagert sind, und
- Denaturieren der Duplexe, gefolgt von langsamem Abkühlen der denaturierten Duplexe bei fortlaufender Messung der Fluoreszenz des Fluorophors der Sonde oder langsamem Erhitzen der Duplexe bei fortlaufender Messung der Fluoreszenz des Fluorophors der Sonde, wobei die Abnahme der Fluoreszenzintensität bzw. die Zunahme der Fluoreszenzintensität Informationen darüber liefert, ob eine spezifische, vollständig komplementäre Short Tandem Repeat in der Probe vorliegt oder nicht.

6. Verfahren zur Genotypisierung nach Anspruch 5, wobei das Amplifizieren von DNA in der Probe mit einer asymmetrischen PCR vorgenommen wird, um amplifizierte, einzelsträngige DNA-Sequenzen zu erhalten.

7. Verfahren zur Genotypisierung nach Anspruch 5, wobei das Amplifizieren von DNA in der Probe mit einer symmetrischen PCR unter Verwendung mit Biotin markierter Primer oder einer anschließenden Lambda-Exonuklease-Verdauung vorgenommen wird, um amplifizierte, einzelsträngige DNA-Sequenzen zu erhalten.

8. Verfahren nach Anspruch 5 bis 7, wobei die Sonden in Lösung oder immobilisiert an einem Träger addiert werden.

## Revendications

1. Pluralité de sondes représentant la variabilité allélique d'un certain locus de répétition en tandem courte à l'intérieur d'une population constituée, dans laquelle chaque sonde est constituée, de 5' à 3' ou de 3' à 5', de :
1) une première région flanquante qui comprend des nucléotides et qui s'annelle avec une région directement à proximité de la séquence d'ADN spécifique d'intérêt et qui contient plus de nucléotides que la deuxième région flanquante, 2) une séquence d'ADN spécifique d'intérêt qui comprend au moins 1 répétition en tandem courte et qui s'annelle avec la région de répétition en tandem courte à l'intérieur de l'échantillon, et 3) une deuxième région flanquante qui comprend au moins 1 nucléotide et qui contient au moins un fluorophore et, ledit fluorophore étant fixé à un résidu de ladite deuxième région flanquante dans la position directement complémentaire à un nucléotide spécifique capable de l'extinction dudit fluorophore d'une manière efficace dudit échantillon, lors de l'hybridation de ladite deuxième région flanquante à l'intérieur de l'échantillon, et ledit nucléotide spécifique capable de l'extinction dudit fluorophore d'une manière efficace étant une guanosine, et, ledit fluorophore étant choisi dans la liste comprenant fluorescéine (FAM), hexachlorofluorescéine (HEX), tétrachloro-6-carboxyfluorescéine (TET), 2,7-diméthoxy-4,5-dichloro-6-carboxyfluorescéine (JOE) ou 6-carboxytétraméthylrhodamine (TAMRA).

2. Pluralité de sondes selon la revendication 1, ledit fluorophore étant fixé à un résidu de cytosine de ladite deuxième région flanquante.

3. Pluralité de sondes selon les revendications 1 ou 2, lesdits nucléotides étant des analogues d'acides nucléiques.

4. Pluralité de sondes selon l'une quelconque des revendications 1 à 3 qui est immobilisée sur un support.

5. Procédé pour génotyper des répétitions en tandem courtes à l'intérieur d'un échantillon comprenant les étapes de :
- fourniture d'un échantillon comprenant un ADN,
- amplification de l'ADN à l'intérieur dudit échantillon qui comprend une séquence d'ADN spécifique d'intérêt afin d'obtenir des séquences d'ADN amplifié,
- ajout d'une pluralité de sondes selon les revendications 1 à 4 auxdites séquences d'ADN amplifié pour obtenir des duplex de séquences d'ADN à simple brin annelés à ladite sonde, et
- dénaturation desdits duplex suivie par un refroidissement lent desdits duplex dénaturés tout en mesurant continuellement la fluorescence du fluorophore de ladite sonde, ou, un chauffage lent desdits duplex tout en mesurant continuellement la fluorescence du fluorophore de ladite sonde, la diminution d'intensité de fluorescence ou l'augmentation d'intensité de fluorescence, respectivement, fournissant des informations sur le fait qu'une répétition en tandem courte complètement complémentaire, spécifique est présente, ou non, dans ledit échantillon.

6. Procédé pour génotyper selon la revendication 5, ladite amplification d'ADN à l'intérieur dudit échantillon étant entreprise par une PCR asymétrique afin d'obtenir des séquences d'ADN simple brin, amplifié.

7. Procédé pour génotyper selon la revendication 5, ladite amplification d'ADN à l'intérieur dudit échantillon étant entreprise par une PCR symétrique en utilisant des amorces marquées par une biotine ou une digestion par une exonucléase lambda subséquente afin d'obtenir des séquences d'ADN simple brin, amplifié.

8. Procédé selon les revendications 5 à 7, lesdites sondes étant ajoutées en solution ou immobilisées sur un support.
